# EUROPÄISCHE PATENTSCHRIFT

(11) **EP 1 146 851 B1**
(45) Veröffentlichungstag und Bekanntmachung des Hinweises auf die Patenterteilung: **12.04.2006**
(21) Anmeldenummer: 00901101.6
(22) Anmeldetag: 14.01.2000
(51) Int. Cl.: A61K 8/00, A61K 8/40, A61K 8/49, A61K 31/415

(54) **BIOTIN ENTHALTENDE GALENISCHE FORMULIERUNG**
GALENIC FORMULATION CONTAINING BIOTIN
FORMULATION GALENIQUE A BASE DE BIOTINE

(30) Priorität: 28.01.1999 DE 19903241
(43) Veröffentlichungstag der Anmeldung: 24.10.2001
(73) Patentinhaber: Merck Patent GmbH, 64293 Darmstadt (DE)
(72) Erfinder: BÜNGER, Joachim, D-64823 Gross-Umstadt-Heubach (DE); DRILLER, Hansjürgen, D-64823 Gross-Umstadt (DE); WOHLRAB, Wolfgang, D-06110 Halle (DE); MOTITSCHKE, Lothar, D-40723 Hilden (DE); HUSCHKA, Christoph, D-06110 Halle (DE); NEUBERT, Reinhard, D-06120 Halle (DE)
(86) Internationale Anmeldenummer: PCT/EP2000/000248
(87) Internationale Veröffentlichungsnummer: WO 2000/044342

(56) Entgegenhaltungen:
- EP-A- 0 222 664
- US-A- 5 166 168
- US-A- 5 710 177
- WILLIAMS A C ET AL: "UREA ANALOGUES IN PROPYLENE GLYCOL AS PENETRATION ENHANCERS IN HUMAN SKIN" INTERNATIONAL JOURNAL OF PHARMACEUTICS,NL,AMSTERDAM, Bd. 56, Nr. 1, 1. November 1989 (1989-11-01), Seiten 43-50, XP000645464 ISSN: 0378-5173

## Beschreibung

Die Erfindung betrifft topisch anwendbare galenische Formulierungen, die eine oder mehrere Verbindungen ausgewählt aus Biotin, den physiologisch verträglichen Salzen von Biotin, Biotinestern und den stereoisomeren Formen dieser Verbindungen und eine oder mehrere Verbindungen ausgewählt aus Harnstoff und Harstoffderivaten enthalten und zur kosmetischen oder medizinischen Behandlung von Haut, Haaren und/oder Nägeln, wie z.B. zur Behandlung von Erkrankungen oder Funktionsstörungen menschlicher oder tierischer Haut, Haare und/oder Nägel geeignet sind.

Der chemische Name für Biotin ist Hexahydro-2-oxothieno[3,4-d]imidazol-4-valeriansäure. Seine chemische Struktur entspricht der Formel

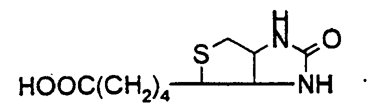

Die Wirksamkeit von Biotin bei Haut-, Haar- und Nagelerkrankungen wurde in der Vergangenheit unterschiedlich bewertet. Obwohl durch eine kontinuierliche Biotintherapie die Wirksamkeit bei weichen, brüchigen und splitternden Nägeln, unterschiedlichen Formen der Alopezie sowie atopischer und seborrhoischer Dermatitis nachweislich aufgezeigt werden konnte, blieb die Anwendung zurückhaltend, da diese trotz keinerlei Nebenwirkungen an die Notwendigkeit eines ausgedehnten Behandlungszeitraums von mehreren Monaten gebunden ist (R. Bitsch et al. (1994) Biotin. Wissenschaftliche Verlagsgesellschaft mbH, Stuttgart; V. E. Colombo et al. (1990) Treatment of brittle fingernails and onychoschizia with biotin: scanning electron microscopy. J. Am. Acad. Dermatol. 23: 1127-1132; G. L. Floersheim (1989) Behandlung brüchiger Fingernägel mit Biotin. Z. Hautkr. 64: 41-48; G. L. Floersheim (1992) Prüfung der Wirkung von Biotin auf Haarausfall und Haarqualität. Z. Hautkr. 67: 246-255; W. Gehring (1996) Der Einfluß von Biotin bei reduzierter Nagelqualität. Eine plazebokontrollierte doppelblinde klinische Studie. Akt. Dermatol. 22: 20-25; U. Siebert et al. (1996) Zur Dosierung und Wirkung von Biotin bei Nagel- und Haarwachstumsstörungen. Zeitschrift Hautnah 6: 438-443).

Es ist bekannt, daß topisch applizierte biotinhaltige Formulierungen mit einem Biotingehalt von 0,25 % und 0,50 % zu einer sichtbaren Reduktion der Fältchenausprägung an der Altershaut führen (L. Gilli et al. (1995) Beeinflussung der Fältchenausprägung bei Altershaut durch topisch appliziertes Biotin. Z. Hautkr. 70 (6): 419-425). Bei diesen Untersuchungen konnten keinerlei epitheliale und histopathologische Veränderungen der behandelten Areale aufgezeigt werden.

Ungeachtet der positiven klinischen Erfahrungen mit Biotin ist die Kenntnis über den Wirkungsmechanismus unvollständig. Der bisherige Wissensstand weist Biotin als Coenzym einer Reihe von Carboxylasen aus, die in verschiedene lebenswichtige Stoffwechselfunktionen eingebunden sind (R. Bitsch et al. (1994) Biotin. Wissenschaftliche Verlagsgesellschaft mbH, Stuttgart). Neben seiner Funktion als Coenzym sind die eigenständigen pharmakologischen Effekte des Biotins, z.B. die Beeinflussung der Keratinstruktur, für den therapeutischen Einsatz von Interesse. Der zugrundeliegende Mechanismus des Einflusses auf das Wachstums- und Differenzierungsverhalten der Keratinozyten wird jedoch kontrovers diskutiert (A. Fritsche (1990) Biotin verändert das Zytokeratinmuster von kultivierten Keratinozyten. Inauguraldissertation., Universität Zürich; A. Fritsche et al. (1991) Pharmakologische Wirkungen von Biotin auf Epidermiszellen. Schweiz. Arch. Tierheilk. 133: 277-283; A. Limat et al. (1996) Proliferation and differentiation of cultured human follicular keratinocytes are not influenced by biotin. Arch. Dermatol. Res. 288: 31-38).

Bedingt durch seine hydrophilen Eigenschaften ist Biotin zu den Stoffen zu zählen, für welches das Stratum corneum mit seiner Lipophilie und der geringen Hydratation im Normalzustand eine Penetrationsbarriere von besonderer Dimension darstellt.

Für die topische Applikation von Biotin existieren somit besondere galenische Anforderungen.

Somit bestand die Aufgabe, topisch anwendbare galenische Formulierungen zur Verfügung zu stellen, die Verbindungen ausgewählt aus Biotin, den physiologisch verträglichen Salzen von Biotin, Biotinestern und den stereoisomeren Formen dieser Verbindungen enthalten und zur kosmetischen oder medizinischen Behandlung von Haut, Haaren und/oder Nägeln geeignet sind.

Überraschend wurde nun gefunden, daß diese Aufgabe durch die Bereitstellung von galenischen Formulierungen enthaltend
a) eine oder mehrere Verbindungen ausgewählt aus Biotin, den physiologisch verträglichen Salzen von Biotin, Biotinestern und den stereoisomeren Formen dieser Verbindungen
   und
b) eine oder mehrere Verbindungen ausgewählt aus Harnstoff und Harnstoffderivaten
gelöst wird.

Die Erfindung betrifft weiterhin
- die Verwendung von einer oder mehreren Verbindungen ausgewählt aus Biotin, den physiologisch verträglichen Salzen von Biotin, Biotinestern und den stereoisomeren Formen dieser Verbindungen und von einer oder mehreren Verbindungen ausgewählt aus Harnstoff und Harnstoffderivaten zur Herstellung einer topisch anwendbaren galenischen Formulierung,
- ein Verfahren zur Herstellung einer topisch anwendbaren galenischen Formulierung, dadurch gekennzeichnet, daß man eine oder mehrere Verbindungen ausgewählt aus Biotin, den physiologisch verträglichen Salzen von Biotin, Biotinestern und den stereoisomeren Formen dieser Verbindungen und eine oder mehrere Verbindungen ausgewählt aus Harnstoff und Harnstoffderivaten gegebenenfalls mit weiteren Wirkstoffen, Hilfs- und/oder Trägerstoffen in eine geeignete Formulierungsform bringt.

Die Anwendung der erfindungsgemäßen galenischen Formulierungen kann sowohl am Menschen als auch an Tieren erfolgen. Die erfindungsgemäßen galenischen Formulierungen können sowohl in der Kosmetik als auch in der Human- und in der Veterinärmedizin angewendet werden. Die Anwendungsgebiete der erfindungsgemäßen galenischen Formulierungen betreffen die Therapie, Prophylaxe und/oder Metaphylaxe von Hauterkrankungen, von Funktionsstörungen der Haut, von Vorgängen der Hautalterung sowie von trockenen Hautzuständen.

Weiterhin betrifft die Erfindung die Verwendung von
a) einer oder mehreren Verbindungen ausgewählt aus Biotin, den physiologisch verträglichen Salzen von Biotin, Biotinestern und den stereoisomeren Formen dieser Verbindungen
   und
b) einer oder mehreren Verbindungen ausgewählt aus Harnstoff und Harnstoffderivaten
zur Herstellung einer topisch anwendbaren galenischen Formulierung
- zur Verbesserung der Penetration der Verbindungen ausgewählt aus Biotin, den physiologisch unbedenklichen Salzen von Biotin, Biotinestern und den stereoisomeren Formen dieser Verbindungen in tiefere Schichten menschlicher oder tierischer Haut, Schleimhaut und/oder Nägel,
- zum Schutz, zur Erhaltung und/oder zum Wiederaufbau der normalen Funktion und Struktur menschlicher oder tierischer Haut und/oder zur Verhinderung umweltbedingter Hautschäden, wie beispielsweise UVbedingter Hautschäden,
- zur Behandlung von Erkrankungen der Haare, Talg- und/oder Schweißdrüsen menschlicher oder tierischer Haut und/oder
- zur kosmetischen Hautbehandlung, insbesondere zur Hautpflege.

Insbesondere bedeutet z.B. "Penetration von Biotin in tiefere Schichten der menschlichen Haut" im Rahmen der vorliegenden Erfindung, daß Biotin durch das Stratum corneum der Human-Haut penetriert. Biotin penetriert ohne einen Penetrationsenhancer nicht bzw, nicht in wirksamer Konzentration durch das Stratum corneum der Human-Haut. Es wurde jedoch z.B. gefunden, daß die Penetration von topisch appliziertem Biotin durch das Stratum corneum in die Human-Haut verbessert wird, wenn Biotin mit einem Penetrationsenhancer, vorzugsweise mit Harnstoff und insbesondere bevorzugt mit Harnstoff in einer Emulsion, kombiniert wird. Es wurde gefunden, daß die Kombination von Biotin mit einem derartigen Penetrationsenhancer beispielsweise die Penetration von Biotin in einer wirksamen Konzentration in die lebende Epidermis ermöglicht.

Als Penetrationsenhancer werden im Rahmen der vorliegenden Erfindung Substanzen bezeichnet, die z.B. die Permeabilität erhöhen bzw. die Penetration der Wirkstoffe am Ort der Anwendung steigern.

Die Anwendung der erfindungsgemäßen galenischen Formulierungen ermöglicht beispielsweise die Penetration von Biotin in ausreichender Konzentration von mindestens 10⁻⁵ mol/l zur Behandlung von Erkrankungen der Haare, Talg- und/oder Schweißdrüsen der Haut. Auch die Anwendung der erfindungsgemäßen galenischen Formulierungen auf Hand- und/oder Fußnägel ermöglicht z.B. die Penetration von Biotin in einer Konzentration von mindestens 10⁻⁵ mol/l in die liefen Nagelanteile und/oder die Nagelmatrix. Üblicherweise wird z.B. etwa 10 min. bis 24 h nach topischer Applikation der erfindungsgemäßen galenischen Formulierung im Stratum spinosum und/oder im Stratum basale der Haut eine Biotinkonzentration erreicht, welche zur Stimulation der epidermalen Lipidsynthese geeignet ist und mindestens 10⁻⁵ mol/l beträgt.

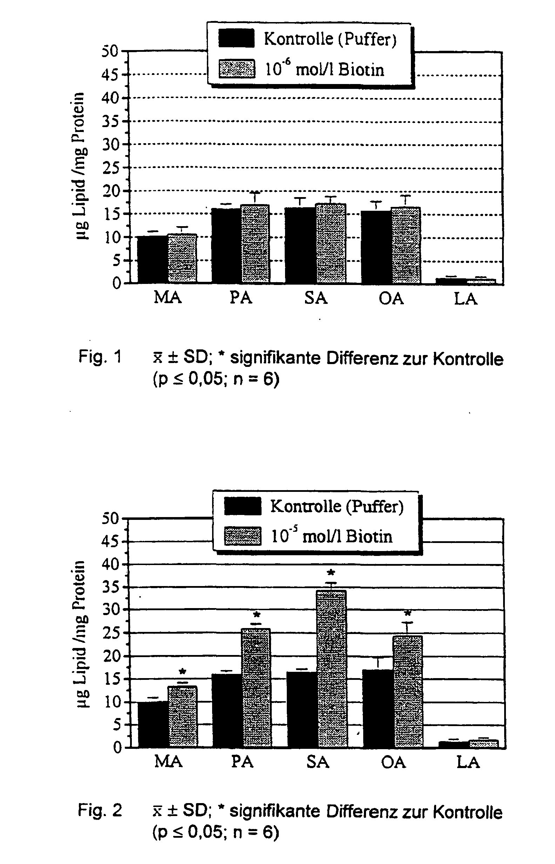

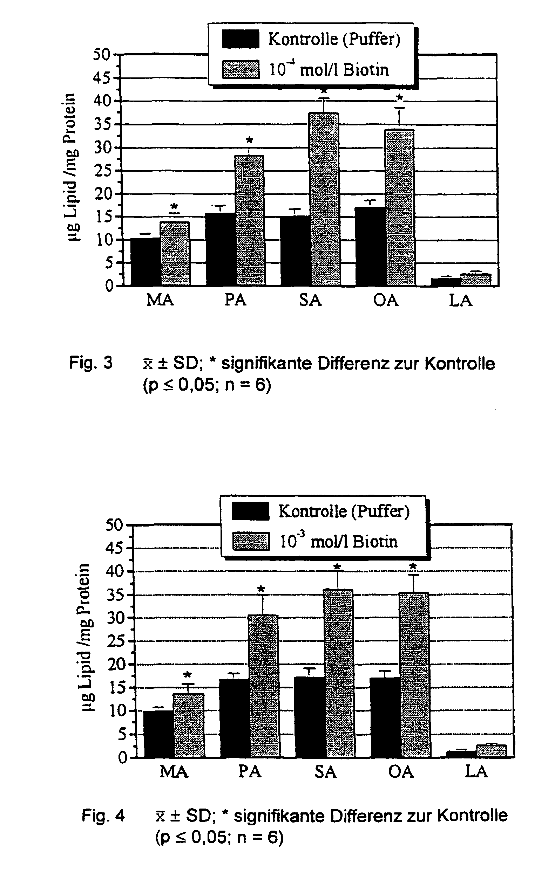

Zudem wurde gefunden, daß der Einfluß von Biotin auf die Lipidsynthese konzentrationsabhängig ist. Dieses Ergebnis ist in den Abbildungen 1 bis 4 dargestellt.

Während niedrige Biotinkonzentrationen im Medium (10⁻⁶ mol/l) als unwirksam eingestuft werden können, ließ sich bei höheren Biotinkonzentrationen im Medium (10⁻⁵ bis 10⁻³ mol/l) die Lipidsyntheseleistung von Keratinozyten steigern.

Steigerungen der Konzentration an den Fettsäuren Myristinsäure (MA), Palmitinsäure (PA), Stearinsäure (SA) und Ölsäure (OA) unter Biotineinwirkung konnten sowohl in der Kultur von HaCaT-Keratinozyten, als auch bei nativen humanen Keratinozyten nachgewiesen werden. Das Ausbleiben einer Steigerung der Konzentration an Linolsäure (LA) ist in Verbindung mit der Unfähigkeit der Keratinozyten, mehrfach ungesättigte Fettsäuren eigenständig zu synthetisieren, zu betrachten. In den Abbildungen 1 bis 4 sind die Ergebnisse für die Biotineinwirkung auf humane kultivierte HaCaT-Keratinozyten dargestellt.

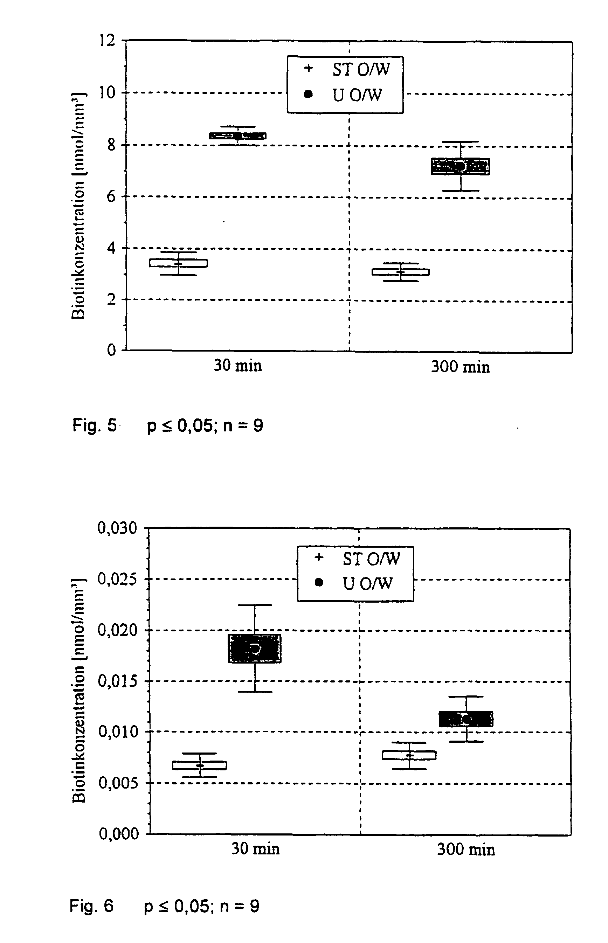

Weiterhin wurde gefunden, daß die Penetration von Biotin in die humane Haut vehikelabhängig ist, wobei die Penetration aus den W/O-Vehikeln deutlich geringer als die Penetration aus den hydrophileren O/W-Vehikeln ist. Allerdings sind weder Standard-W/O- noch Standard-O/W-Vehikel, die keinen Penetrationshancer enthalten, dazu geeignet, ausreichend hohe epidermale Biotinkonzentrationen für eine Stimulation der Lipidsynthese zu erzielen. Die ungenügende Penetration von Biotin aus einem Standard-O/W-Vehikel, der keinen Penetrationshancer enthält, ist für das Stratum corneum in Abbildung 5 und die Epidermis in Abbildung 6 gezeigt (s. Werte für ST O/W in den Abbildungen 5 und 6).

Eine Vehikeloptimierung wurde erfindungsgemäß durch die Zugabe von Harnstoff in die Standardvehikel erreicht. Durch die Einarbeitung von Harnstoff in die Standardvehikel resultieren erfindungsgemäße Formulierungen, die eine gesteigerte Penetration von Biotin in die humane Haut bewirken. Die Penetrationssteigerung ist im Ausmaß von dem verwendeten Vehikel abhängig.

Beispielsweise führt die Verwendung einer erfindungsgemäßen O/W-Emulsion mit 10 % Harnstoff bereits nach einer kurzen Einwirkdauer von 30 min. zu einer effektiven epidermalen Biotinkonzentration. Der Effekt war auch nach einer prolongonierten Applikationsdauer von 300 min. nachweisbar. Diese Ergebnisse sind ebenfalls in den Abbildungen 5 und 6 dargestellt (s. Werte für U O/W in den Abbildungen 5 und 6).

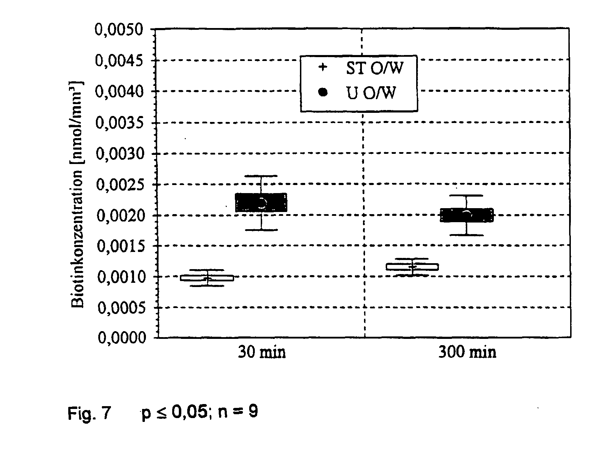

Der Vergleich der Biotinkonzentration in der Dermis nach Penetration von Biotin aus einem Standard-O/W-Vehikel (s. Werte für ST O/W) und nach Penetration von Biotin aus einem entsprechenden 10 Gew.-% Harnstoff enthaltenden Vehikel (s. Werte für U O/W) in die humane Haut ex vivo ist in Abbildung 7 dargestellt.

Die Biotinkonzentration in der Dermis nach Penetration von Biotin aus dem 10 Gew.% Harnrstoff enthaltenden Vehikel in die humane Haut ex vivo liegt sowohl nach 30 min. als auch nach 300 min. deutlich unter 0,01 nmol/mm³. Die Konzentration von 0,01 nmol/mm³ entspricht der wirksamen Biotinkonzentration von 10⁻⁵ mol/l aus Abbildung 2. Dies zeigt, daß durch die topische Applikation von erfindungsgemäßen Formulierungen, die Harnstoff enthalten, die Penetration von Biotin durch das Stratum corneum in vorteilhafter Weise so gesteuert werden kann, daß die wirksame Biotinkonzentration in der Epidermis überschritten wird, aber in der Dermis unterschritten bleibt (s. Abbildungen 6 und 7).

In der US 5,166,168 werden topisch anwendbare Formulierungen beschrieben, die Biotin oder ein pharmazeutisch unbedenkliches Salz von Biotin enthalten und zur Behandlung von Nagelkrankheiten geeignet sind. Diese Formulierungen können Penetrationsenhancer in einer effektiven Menge enthalten, beispielsweise Menthol, Propylenglykol, Dimethylsulfoxid, Dimethylacetamid, Dimethylformamid und Azon. Es gibt jedoch keinen Hinweis auf Harnstoff oder Harnstoffderivate als Penetrationsenhancer.

EP-A-222 664 beschreibt Imidazol-2-on- und Imidazolidin-2-on-Derivate, welche sich als Penetrationsverbesserer für topische Anwendungen eignen.

In US-A-5,710,177 werden kosmetische Zubereitungen beschrieben, die Biotin und ein oder mehrere alpha-Hydroxycarbonsäuren bzw. alpha-Ketocarbonsäuren sowie optional zusätzliche Antioxidantien enthalten.

In A.C. Williams et al. "Urea analogues in propylene glycol as penetration enhancers in human skin" Int. J. Pharm. 56(1), 1989, pp.43-50 werden Harnstoff und verschiedene Harnstoffderivate auf ihre Eignung als Penetrationsverbesserer für den Wirkstoff 5-Fluorouracil untersucht. Es zeigte sich, dass sowohl mit Harnstoff als auch den untersuchten Harnstoffderivaten alleine keine penetrationsverbessemde Wirkung erzielt werden kann. Erst die Kombination mit Propylengylcol ermöglichte eine Penetrationsverbesserung.

Die Verbindungen ausgewählt aus Biotin, den physiologisch verträglichen Salzen von Biotin, Biotinestern und den stereoisomeren Formen dieser

Verbindungen können in den galenischen Zubereitungen z.B. als optische isomere, Diastereomere oder Racemate vorliegen.

Die Biotinester sind durch folgende generische Strukturformel gekennzeichnet:

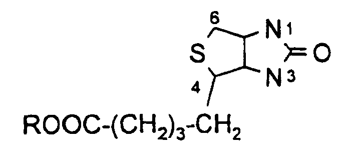

Erfindungsgemäß bedeutet R eine verzweigte oder unverzweigte Alkylgruppe mit 1 bis 18 C-Atomen oder eine verzweigte oder unverzweigte Alkenylgruppe mit 1 bis 18 C-Atomen. Bevorzugte Biotinester sind diejenigen, in denen R Methyl oder Ethyl darstellt.

Unter den Verbindungen ausgewählt aus Biotin, den physiologisch verträglichen Salzen von Biotin, Biotinestern und den stereoisomeren Formen dieser Verbindungen ist Biotin bevorzugt, insbesondere D-cis-Hexahydro-2-oxothieno[3,4-d]imidazol-4-valeriansäure der Formel

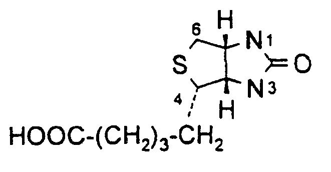

Biotin kann in der Form jedes bekannten pharmazeutisch unbedenklichen Salzes in den erfindungsgemäßen Formulierungen verwendet werden. Bevorzugte physiologisch verträgliche Salze von Biotin sind beispielsweise Alkali-, Erdalkali- oder Ammoniumsalze, wie Na-, K-, Mg- oder Ca-Salze, sowie Salze abgeleitet von organischen Basen wie z.B. Ethylamin, Triethylamin, Ethanolamin, Diethylaminoethanol, Ethylendiamin, Piperidin, Morpholin, 2-Piperidinoethanol, Benzylamin und Procain.

Geeignete Harnstoffderivate sind solche, die nach Spaltung Harnstoff freisetzen. Unter den Harnstoffderivaten ist Allantoin bevorzugt.

Unter den Verbindungen ausgewählt aus Harnstoff und den Harnstoffderivaten ist Harnstoff bevorzugt.

Unter den erfindungsgemäßen galenischen Formulierungen sind somit diejenigen bevorzugt, die Biotin und/oder Harnstoff enthalten.

Die Verbindungen ausgewählt aus Harnstoff und Harnstoffderivaten können in unterschiedlichen galenischen Formulierungen, wie z.B. in O/W-oder W/O-Emulsionen, eingearbeitet sein. Vorzugsweise ist Harnstoff in O/W-Emulsionen eingearbeitet.

Die Herstellung der galenischen Formulierung erfolgt, indem eine oder mehrere Verbindungen ausgewählt aus Biotin, den physiologisch verträglichen Salzen von Biotin, Biotinestern und den stereoisomeren Formen dieser Verbindungen und eine oder mehrere Verbindungen ausgewählt aus Harnstoff und Harnstoffderivaten gegebenenfalls mit Hilfs-und/oder Trägerstoffen in eine geeignete Formulierungsform gebracht werden. Die Hilfs- und Trägerstoffe stammen aus der Gruppe der Trägermittel, Konservierungsstoffe und anderer üblicher Hilfsstoffe.

Die galenischen Formulierungen auf der Grundlage einer oder mehrerer Verbindungen ausgewählt aus Biotin, den physiologisch verträglichen Salzen von Biotin, Biotinestern und den stereoisomeren Formen dieser Verbindungen und einer oder mehrerer Verbindungen ausgewählt aus Harnstoff und Harnstoffderivaten werden äußerlich angewendet.

Als Anwendungsform seien z.B. genannt: Lösungen, Suspensionen, Emulsionen, Pasten, Salben, Gele, Cremes, Lotionen, Lacke, Puder, Seifen, tensidhaltige Reinigungspräparate, Öle und Sprays. Zusätzlich zu einer oder mehreren Verbindungen ausgewählt aus Biotin, den physiologisch verträglichen Salzen von Biotin, Biotinestern und den stereoisomeren Formen dieser Verbindungen und einer oder mehrerer Verbindungen ausgewählt aus Harnstoff und Harnstoffderivaten werden der Formulierung beliebige übliche Trägerstoffe, Hilfsstoffe und gegebenenfalls weitere Wirkstoffe zugesetzt.

Die gegebenenfalls in den erfindungsgemäßen galenischen Formulierungen enthaltenen Hilfsstoffe und Wirkstoffe können sowohl hydrophiler als aus lipophiler Art sein.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können neben einer oder mehreren Verbindungen ausgewählt aus Biotin, den physiologisch verträglichen Salzen von Biotin, Biotinestern und den stereoisomeren Formen dieser Verbindungen und einer oder mehrerer Verbindungen ausgewählt aus Harnstoff und Harnstoffderivaten die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können neben einer oder mehreren Verbindungen ausgewählt aus Biotin, den physiologisch verträglichen Salzen von Biotin, Biotinestern und den stereoisomeren Formen dieser Verbindungen und einer oder einer oder mehreren Verbindungen ausgewählt aus Harnstoff und Harnstoffderivaten die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können neben einer oder mehreren Verbindungen ausgewählt aus Biotin, den physiologisch verträglichen Salzen von Biotin, Biotinestern und den stereoisomeren Formen dieser Verbindungen und einer oder mehrerer Verbindungen ausgewählt aus Harnstoff und Harnstoffderivaten die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können neben einer oder mehreren Verbindungen ausgewählt aus Biotin, den physiologisch verträglichen Salzen von Biotin, Biotinestern und den stereoisomeren Formen dieser Verbindungen und einer oder mehrerer Verbindungen ausgewählt aus Harnstoff und Harnstoffderivaten die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können neben einer oder mehreren Verbindungen ausgewählt aus Biotin, den physiologisch verträglichen Salzen von Biotin, Biotinestern und den stereoisomeren Formen dieser Verbindungen und einer oder mehrerer Verbindungen ausgewählt aus Harnstoff und Harnstoffderivaten die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können neben einer oder mehreren Verbindungen ausgewählt aus Biotin, den physiologisch verträglichen Salzen von Biotin, Biotinestern und den stereoisomeren Formen dieser Verbindungen und einer oder mehrerer Verbindungen ausgewählt aus Harnstoff und Harnstoffderivaten die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können neben einer oder mehrerer Verbindungen ausgewählt aus Biotin, den physiologisch verträglichen Salzen von Biotin, Biotinestern und den stereoisomeren Formen dieser Verbindungen und einer oder mehreren Verbindungen ausgewählt aus Harnstoff und Harnstoffderivaten die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Vorzugsweise liegen die Verbindungen ausgewählt aus Biotin, den physiologisch verträglichen Salzen von Biotin, Biotinestern und den stereoisomeren Formen dieser Verbindungen, die Penetrationsenhancer bzw. -promotoren und/oder die Hilfsstoffe in den erfindungsgemäßen galenischen Formulierungen in kolloidalen Trägersystemen eingearbeitet vor, insbesondere liegen sie in Nanopartikeln, Liposomen oder Mikroemulsionen eingearbeitet vor.

Weitere typisch galenische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Bevorzugte Anwendungsformen sind Pasten, Salben, Cremes, Emulsionen, Gele und Lacke. Hierbei liegt die erfindungsgemäße galenische Formulierung besonders bevorzugt als O/W-Emulsion vor. Die Applikation auf Hand- und/oder Fußnägel erfolgt vorzugsweise als Lack.

Der Anteil an der oder an den Verbindungen ausgewählt aus Biotin, den physiologisch verträglichen Salzen von Biotin, Biotinestern und den stereoisomeren Formen dieser Verbindungen in der erfindungsgemäßen galenischen Formulierung beträgt vorzugsweise von 0,05 bis 5 Gew.% bezogen auf die gesamte galenische Formulierung.

Der Anteil an der oder an den Verbindungen ausgewählt aus Harnstoff und Harnstoffderivaten in der erfindungsgemäßen galenischen Formulierung beträgt vorzugsweise von 1 bis 40 Gew.% bezogen auf die gesamte galenische Formulierung.

Vorzugsweise enthalten die galenischen Formulierungen neben einer oder mehreren Verbindungen ausgewählt aus Biotin, den physiologisch verträglichen Salzen von Biotin, Biotinestern und den stereoisomeren Formen dieser Verbindungen und einer oder mehreren Verbindungen ausgewählt aus Harnstoff und Harnstoffderivaten eine oder mehrere Substanzen mit Radikalfängereigenschaften, insbesondere Vitamin E, Vitamin C und/oder deren Ester. Der Anteil an der oder an den Verbindungen mit Radikalfängereigenschaften beträgt vorzugsweise von 0,01 bis 30 Gew.%, besonders bevorzugt von 1 bis 30 Gew.% bezogen auf die gesamte galenische Formulierung.

Alle Verbindungen oder Komponenten, die in den galenischen Formulierungen verwendet werden können, sind entweder bekannt und käuflich erwerbbar oder können nach bekannten Methoden synthetisiert werden.

Die folgenden Beispiele dienen zur Verdeutlichung der Erfindung und sind keinesfalls als Limitierung aufzufassen. Alle %-Angaben sind, soweit nichts anderes angegeben ist, Gewichtsprozent.

Folgende Verbindungen oder Komponenten wurden in den Beispielen 1 und 2 verwendet:

| | |
|---|---|
| Biotin | Carl Roth GmbH, Karlsruhe, BRD |
| ¹⁴C-Biotin (spez. Akt. 55 | Amersham-Buchler GmbH, |
| mCi/mmol) | Braunschweig, BRD |
| Chloroform reinst p.a. | Merck KGaA, Darmstadt, BRD |
| Harnstoff | Merck KGaA, Darmstadt, BRD |
| Kohlendioxidgas | Messer Griesheim, Leipzig, BRD |
| Lanette® N | Universitätsapotheke der Martin-Luther-Universität Halle Wittenberg |
| Methanol reinst p.a. | Laborchemie Apolda GmbH, BRD |
| Methanol gradient grade für die HPLC | Merck KGaA, Darmstadt, BRD |
| Natriumchlorid (NaCl) reinst p.a. | Merck KGaA, Darmstadt, BRD |
| Paraffinum subliquidum | Carl Roth GmbH, Karlsruhe, BRD |
| POPOP | Canberra Packard GmbH, Dreieich, BRD |
| PPO | Canberra Packard GmbH, Dreieich, BRD |
| Protein Assay Kit P5656 | Sigma-Aldrich Chemie GmbH, Deisenhofen, BRD |
| Serum free keratinocyte medium | (inklusive Gibco BRL Life Technologies GmbH, Eggenstein, rEGF+BPE) BRD |
| Soluene 350 | Canberra Packard GmbH, Dreieich, BRD |
| Tesafilm | Beiersdorf AG, Hamburg, BRD |
| Toluol | Merck-Schuchardt, Hohenbrunn, BRD |
| Trimethylsulfoniumhydroxid | Macherey Nagel GmbH, Düren, BRD |
| Vaselinum album | Carl Roth GmbH, Karlsruhe, BRD |

Die INCI-Namen verwendeter Rohstoffe sind wie folgt:

| Rohstoff | INCl |
|---|---|
| Lanette® N | Emulgierender Cetylstearylalkohol DAB 10 |
| Paraffin | Mineralöl |
| Arlamol HD | isohexadecan |
| Mirasil DM 350 | Dimethicon |
| Lanette® O | Cetearylalkohol |
| Span 60 | Sorbitanstearat |
| Montanov 68 | Cetearylalkohol (und) Cetearylglukosid |
| Rhodicare S | Xanthan Gum |
| Arlacel 165 V | Glycerylstearat (und) PEG-100 Stearat |
| Germaben II-E | Propylenglykol (und) Diazolidinylharnstoff (und) Methylparaben (und) Propylparaben |
| lsolan PDI | Diisostearoyl Polyglyceryl-3 Diisostearat |
| Cutina HR Pulver | Hydriertes Castoröl |

Folgende Geräte und Hilfsmittel wurden in den Beispielen verwendet:

| | |
|---|---|
| Absorptionsreader | Labsystems, iEMS, Merlin, Bornheim-Hersel, BRD |
| Biopsiestanzen | Stiefel Laboratorium GmbH, Offenbach, BRD |
| Flüssigkeitszintillationszähler | Wallac 1410, Berthold, Berlin, BRD |
| Franz-Diffusionszelle | Crown Glass Company, Sommerville, New Jersey, USA |
| mit Wasserbad | Thermostat B3-DC1, Haake, Karlsruhe, BRD |
| GC-Anlage | Finnigan MAT Magnum, GC-Varian 3400, Finnigan-MAT, Bremen, BRD |
| GC-Kapillarsäule | FFAP, Macherey-Nagel, Düren oder Fisher Scientific, Nidderau, BRD |
| Gefriermikrotom | Mikrotom-Modell 1206, Frigomobil Reichert-Jung, Heidelberg-Nußloch, BRD |
| Gewebe-Kulturflaschen | Greiner GmbH, Frickenhausen, BRD |
| HaCaT-Keratiozyten | Deutsches Krebsforschungsinstitut Heidelberg, BRD |
| Präzisionswaagen | Typ 870-13, Gottl. Kern & Sohn, Albstadt 1, BRD |
| Sterilfilter Nalgene 0,2 µm | Nalge-Company, Worcester, USA |
| Szintillationsmeßgefäße | Pony Vial, Pico Glass Vial, Econo Glass Vial, Canberra Packard GmbH, Dreieich, BRD |
| Vakuumrotationsverdampfer | Rotivapor RE 111, Laboratoriumstechnik Büchli, Flawil, Schweiz |
| Mit Wasserbad | Büchli 461, Laboratoriumstechnik Büchli, Flawil, Schweiz |

Der in den Beispielen 1 und 2 verwendete Puffer nach Mc. Iloaine entspricht der Pufferlösung ohne Biotin aus den Beispielen 4 und 5 und wird wie folgt hergestellt: 86 ml 0,2 M Dinatriumhydrogenphosphatmonohydrat-Lösung wird mit 14 ml 0,1 M Zitronensäuremonohydrat-Lösung vermischt (pH: 7,2).

### Beispiel 1

### Untersuchungen zum Lipidgehalt von humanen Keratinozyten

### Durchführung der Untersuchungen zum Lipidstoffwechsel:

HaCaT-Zellen (human adult low calcium high temperature keratinocytes) und native humane Keratinozyten werden in Gibco's Keratinocyte Serum Free Medium (SFM) unter Zusatz von BPE (25 µg/ml Rinderhypophysenextrakt) und rEGF (0,1-0,2 ng/ml, rekombinanter Epidermaler Wachstumsfaktor) in 5%iger CO₂-Atmosphäre (pH 7,2-7,4) bei 36,6 °C im low calcium-Status (0,09 mM) in Kulturflaschen (1 Million Zellen/20 ml SFM) kultiviert und am 3. Tag nach Einsaat mit der Biotinlösung (Biotin gelöst in Puffer nach Mc Iloaine; Mediumkonzentration 10⁻⁷ - 10⁻³ mol/l und Lösungsmittelkontrolle) versetzt. Am 6. Tag erfolgt das Ernten der konfluenten Monolayer und die Probenvorbereitung, wobei einzelne Pellets zu je 3 Millionen Zellen für die Lipidextraktion sowie für die Proteinbestimmung verwendet werden.

### Durchführung der Lipidextraktion:

Die Extraktion der Zellen erfolgt in Anlehnung an die Lipidextraktion von Bligh und Dyer (B.E. Bligh et al. (1959) A rapid method of total lipid extraction and purification. Can. J. Biochem. Physiol. 37: 911-917). Dazu wird das Zellpellet mit 20 ml eines Lösungsmittelgemisches versetzt (Chloroform : Methanol = 2 : 1), unter Rühren homogenisiert und 30 min. im Dunkeln unter Schütteln extrahiert. Anschließend wird über einen Sterilfilter filtriert und der Extrakt am Vakuumrotationsverdampfer eingeengt. Der Lipidfilm wird in 4 ml Methanol (gradient grade für die HPLC) aufgenommen und via GC-MS analysiert.

### Analytik der Fettsäuren mittls GC-MS:

Die extrahierten Lipide der Keratinozyten werden als methanolische Lösungen via GC-MS analysiert, um die darin enthaltenen Fettsäuren zu quantifizieren. 100 µl der Lösung werden mit je 1 µl 0,2 M Trimethylsulfioniumhydroxid zu Methylestern umgesetzt, um die Verdampfbarkeit zu verbessern.

| | |
|---|---|
| Gerät | Finnigan MAT Magnum, GC-Varian 3400 |
| Autosampler | Finnigan MAT A 200 S |
| Detektor (MS) | lontrap |
| lonisierung | Elektronenstoß 70 eV |
| Kapillarsäule | FFAP, 25 m x 0,25 mm x 0,25 µm, mit 10 m unbelegter Vorsäule |
| Injektionsart | splitless, Splitventilöffnung nach 1 min |
| Injektionstemperatur | 250 °C |
| Temperaturprogramm | 60-160 °C : 15 °C/min 160-240 °C : 25 °C/min |
| Quellentemperatur | 220 °C |
| Trägergas | Helium, Vordruck 13 psi |
| Flußrate | 40 cm/min |
| Kalibrierung | Externer Standard (Konzentrationen: 0,02 -10 µg/ml) |

### Bestimmung des Proteingehaltes:

Der Proteingehalt der Zellen wird mit dem Protein Assay Kit nach der Lowry-Methode bestimmt. Nach der Präparation laut Vorschrift zum Kit (P 5656) erfolgte die Messung am Absorptionsreader bei 690 nm.

Die Ergebnisse des Einflusses von Biotin auf den Fettsäuregehalt von HaCaT-Keratinozyten sind für verschiedene Konzentrationen von Biotin (gelöst in Puffer nach Mc Iloaine) in den Abbildungen 1 bis 4 dargestellt. Als Kontrolle ist in den Abbildungen 1 bis 4 zudem der Fettsäuregehalt der HaCaT-Keratinozyten nach entsprechender Einwirkung des Puffers ohne Biotin dargestellt. Die Gehalte der Fettsäuren Myristinsäure (MA), Palmitinsäure (PA), Stearinsäure (SA), Ölsäure (OS) und Linolsäure (LA) sind bezogen auf den Proteingehalt der Zellen dargestellt.

### Beispiel 2

### Penetrationsuntersuchungen an humaner Haut ex vivo

### Modellbeschreibung und Versuchsbedingungen:

Zur Charakterisierung des Penetrationsverhaltens von Biotin aus unterschiedlichen Emulsionen in humane exzidierte Haut, wird als in vitro-Modell die FRANZ'sche Diffusionszelle gewählt. Die Auswahl des Modells stützt sich auf die Erfahrungen der Autoren, die korrelierende Ergebnisse zwischen den in vitro-Daten des Modells und den in vivo-Resultaten feststellen konnten (R.J. Feldmann, et al. (1970): Absorption of some organic compounds through the skin in man. J. Invest. Dermatol. 54: 399-404; T.J. Franz (1975): Percutaneous absorption. On the relevance of in vitro data. J. Invest. Dermatol. 64: 190-195).

Der Grundkörper der FRANZ'schen Diffusionszelle besteht aus einem doppelwandigen, temperierbaren Glasteil. Darin befindet sich die durch Rühren ständig im homogenen Zustand gehaltene Akzeptorflüssigkeit. Im direkten Kontakt zum Akzeptor liegt das Probenmaterial auf einer Gaze, die durch einen Glasring und eine Metallklammer stabilisiert wird. Eine Glasbedeckung fungiert als Verdunstungsschutz zur Einhaltung weitgehend konstanter Hydratationsbedingungen während des Untersuchungszeitraumes.

Durch den Einsatz von Wasser als Akzeptormedium werden bei der einmaligen Applikation der zu untersuchenden Vehikel *sink*-Bedingungen für den hydrophilen Wirkstoff realisiert. Zur Standardisierung der Versuchsbedingungen kann auf Probandenmaterial mit geringer Schwankung des Alters zurückgegriffen werden.

Um Informationen über das Penetrationsausmaß des Wirkstoffes im Kurzzeit- als auch im Langzeitbereich zu erhalten, erfolgt die Datenaufnahme nach Einwirkzeiten von 30 und 300 min.

Aus folgenden Komponenten wird eine erfindungsgemäße O/W-Emulsion (wasserhaltige hydrophile Salbe) enthaltend Biotin und Harnstoff hergestellt:

| | | Gew.-% |
|---|---|---|
| B | Lanette N | (1) 9.0 |
| | Paraffinum subliquidum | (2) 10.5 |
| | Vaselinum album | (2) 10.5 |
| A | Wasser, demineralisiert und Puffer nach Mc Iloaine (Gew.-Verhältnis = 50 : 50) | ad 100 |
| | Biotin | (2) 0.1 |
| | Harnstoff | (3) 10.0 |

### Herstellung:

Der Wirkstoff Biotin wird zunächst im Puffer nach Mc Iloaine gelöst und zusammen mit dem Harnstoff (trifft nur bei der harnstoffhaltigen erfindungsgemäßen Formulierung zu) in demineralisiertem Wasser verteilt.
Anschließend erfolgt die anteilmäßige Einarbeitung der Wasserphase B in die aufgeschmolzene lipohile Phase A der restlichen Bestandteile der Emulsion. Das System wird bis zum Erkalten gerührt.

### Bezugsquellen:

(1) Universitätsapotheke der Martin-Luther-Universität Halle-Wittenberg
(2) Carl Roth GmbH, Karlsruhe
(3) Merck KGaA, Darmstadt

### Präparation des biologischen Materials:

Die für die Penetrationsstudien verwendeten Hautpräparate stammen von Mammareduktionsplastiken. Das Untersuchungsmaterial wird innerhalb von 2 h - 4 h nach der Operation aufgearbeitet. Dazu erfolgt zunächst die Säuberung mit isotonischer NaCl-Lösung. Es schließt sich das mechanische Entfemen des subkutanen Fettgewebes und die Herstellung runder Stücke mit Hilfe eines Locheisens (d = 2 cm) an, so daß danach einzeln in Aluminiumfolie verpackte Präparate bei -20 °C im Tiefkühlschrank für maximal drei Monate gelagert werden können.

Das Alter der Probandinnen lag zwischen 17 und 53 Jahren, wobei bei der Versuchsplanung aus Standardisierungsgründen versucht wird, weitgehend auf altersgleiche Haut zurückzugreifen.

### Versuche mit radioaktiv markierten Substanzen:

Zirka 30 min. vor Versuchsbeginn werden die FRANZ'schen Diffusionszellen mit dem Akzeptormedium Wasser gefüllt und auf 32 °C temperiert. Unmittelbar vor der Vehikelapplikation wird diese mit dem in Ethanol gelösten Tracer ¹⁴C-Biotin (5 µCi) versetzt. Dabei ist Wert auf homogene Vermischung mit der unmarkierten Zubereitung sowie auf die vollständige Verdunstung des Lösungsmittels zu legen. Anschließend erfolgt der homogene Antrag von ca. 20 mg Emulsion auf der gesamten Epidermisfläche eines aufgetauten und mit einem Watteträger vom Gefrierwasser getrockneten Hautstückes. Unmittelbar nach der Vehikelapplikation wird dieses auf eine Gaze gelegt und in der Diffusionszelle fixiert.

Nach Ablauf der Versuchszeiten von 30 min. und 300 min. wird die Haut dem Modell entnommen und der Formulierungsrückstand vorsichtig mit Watteträgern entfemt. Die weitere Aufarbeitung erfordert die Fixiemg des Untersuchungsmaterials mit Stecknadeln an der Peripherie auf einer mit Aluminiumfolie bespannten Polystyrenunterlage. Unter Zuhilfenahme einer Schablone kann das Stratum corneum reproduzierbar auf einer Kreisfläche (d = 1,6 cm) durch 20 Tesaflmabrisse vollständig eliminiert werden. Dessen quantitative Bestimmung erfolgt in paarweise Fraktionierung der Abrisse (10 x 2). Aus der lebenden Haut werden je drei Stanzbiopsien (d = 0,6 cm) entnommen, die zur Anfertigung von Horizontalschnitten (8 x 20 µm - Epidermis, 30 x 40 µm - Dermis) an einem Gefriermikrotom dienen. Für die Quantifizierung der Proben werden diese mit Methanol und Szintillator [4,0 g PPO (2,5-Diphenyloxazol) und 0,1 g POPOP 1,4-Di-2-(5-phenyloxazolyl)-benzen in 1,0 l Toluol)] versetzt, wobei zuvor die Epidermis- und Dermisschnitte sowie die Stanzenreste zur vollständigen Zersetzung des Gewebes über Nacht in 100 µl bzw. 1 ml Soluene im Szintillationsmeßgefäß belassen werden. Im Anschluß daran werden die Proben unter Mitführung zweier Leerwerte im Flüssigkeitsszintillationszähler vermessen.

Die Ergebnisse für die Penetration von Biotin aus der erfindungsgemäßen O/W-Salbe dieses Beispiels enthaltend 10 Gew.% Harnstoff (s. Werte für U O/W) sind für Einwirkzeiten von 30 min. und 300 min. in Abbildung 5 für das Stratum corneum, in Abbildung 6 für die Epidermis und in Abbildung 7 für die Dermis dargestellt. Als Vergleich sind in den Abbildungen 5 bis 7 zudem die Ergebnisse für die Penetration von Biotin aus einer entsprechend der erfindungsgemäßen O/W-Salbe hergestellten Salbe, in der jedoch Harnstoff durch eine Lösung bestehend aus 50 Gew.% demineralisiertem Wasser und 50 Gew.% Puffer nach Mc Iloaine ersetzt ist (s. Werte für ST O/W), dargestellt. Die Ergebnisse sind als Konzentration (nmol Biotin pro mm³ Stratum comeum, Epidermis oder Dermis) dargestellt.

### Beispiel 3

Aus folgenden Komponenten wird eine erfindungsgemäße Hand- und Nagelcreme enthaltend Biotin und Harnstoff hergestellt:

| | | | Gew.-% |
|---|---|---|---|
| A | Paraffin | (Art.-Nr.107162) | (1) 2.0 |
| | Arlamol HD | | (2) 2.0 |
| | Isopropylpalmitat | | (3) 3.0 |
| | Sojaöl | | (4) 0.5 |
| | Mirasil DM 350 | | (5) 1.0 |
| | Lanette O | | (3) 1.0 |
| | Span 60 | | (2) 1.5 |
| | Montanov 68 | | (6) 4.0 |
| | (-)-α-Bisabolol | (Art.-Nr. 130170) | (1) 0.3 |
| B | Wasser, demineralisiert | | ad 100 |
| | Glycerin, 87%ig | (Art.-Nr. 104091) | (1) 10.0 |
| | D-Panthenol | | (7) 0.5 |
| | D-(+)-Biotin | (Art.-Nr. 130220) | (1) 0.05 |
| | Harnstoff | | (1) 10.0 |
| | Konservierungsmittel | | q.s. |
| C | Rhodicare | | (5) 0.3 |
| D | Parfüm Bianca | | (8) 0.2 |

Als Konservierungsmittel können
0.05 % Propyl-4-hydroxybenzoat (Act.-Nr. 107427),
0.15 % Methyl-4-hydroxybenzoat (Art.-Nr. 106757) oder
0.30 % Germall 115 (ISP, Frechen)
verwendet werden.

### Herstellung:

Die vereinigten Phasen A und B werden getrennt auf 75 °C erhitzt. Danach wird die Phase C bei 75 °C unter Rühren langsam zur Phase B gegeben und gerührt bis eine homogene Mischung entsteht. Anschließend wird Phase A zugegeben und bis zur Homogenität gerührt. Es wird unter Rühren abkühlen gelassen und das Parfüm bei ca. 35 °C zugegeben.

### Bezugsquellen:

- (1): Merck KGaA, Darmstadt
- (2): ICI Surfactants, Essen
- (3): Henkel KGaA, Düsseldorf
- (4): Gustav Heess, Stuttgart
- (5): Rhodia, Frankfurt
- (6): Seppic, Frankreich
- (7): BASF AG, Ludwigshafen
- (8): H&R, Holzminden

### Beispiel 4

Aus folgenden Komponenten wird eine erfindungsgemäße O/W-Emulsion enthaltend Biotin und Harnstoff hergestellt:

| | | Gew.-% |
|---|---|---|
| A | Paraffin, dickflüssig | (1) 8.0 |
| | Isopropylmyristat | (1) 7.0 |
| | Lanette O | (2) 3.0 |
| | Arlacel 165 V | (3) 5.0 |
| B | Wasser, demineralisiert | ad 100 |
| | Glycerin, 87%ig | (1) 3.0 |
| | Harnstoff | (1) 10.0 |
| | Biotinpufferlösung (1%ig) | (1) 10.0 |
| | Germaben II-E | (4) 0.5 |

### Herstellung:

Die vereinigten Phasen A und B werden getrennt auf 75 °C erhitzt. Danach wird die Phase A unter Rühren langsam zur Phase B gegeben und die resultierende Emulsion homogenisiert. Anschließend wird unter Rühren auf 30 °C abkühlen gelassen.

### Bezugsquellen:

- (1): Merck KGaA, Darmstadt
- (2): Henkel KGaA, Düsseldorf
- (3): ICI
- (4): ISP

### Beispiel 5

Aus folgenden Komponenten wird eine erfindungsgemäße W/O-Emulsion enthaltend Biotin und Harnstoff hergestellt:

| | | Gew.-% |
|---|---|---|
| A | Isolan PDI | (2) 3.0 |
| | Paraffinöl, flüssig | (1) 17.0 |
| | Isopropylmyristat | (1) 5.0 |
| | Wachs, gebleicht | (1) 0.2 |
| | Cutina HR Pulver | (3) 0.3 |
| B | Wasser, demineralisiert | ad 100 |
| | Glycerin, 87%ig | (1) 4.0 |
| | Harnstoff | (1) 10.0 |
| | Biotinpufferlösung (1%ig) | (1) 10.0 |
| | Magnesiumsulfat-Hepta- | (1) 0.5 |
| | hydrat | |
| | Germaben II-E | (4) 0.5 |

### Herstellung:

Die vereinigten Phasen A und B werden getrennt auf 75 °C erhitzt. Danach wird die Phase B unter Rühren langsam zur Phase A gegeben und homogenisiert. Anschließend wird unter Rühren auf Raumtemperatur abkühlen gelassen.

### Bezugsquellen:

- (1): Merck KGaA, Darmstadt
- (2): Th. Goldschmidt
- (3): Henkel KGaA, Düsseldorf
- (4): ISP

## Patentansprüche

1. Galenische Formulierung enthaltend
a) eine oder mehrere Verbindungen ausgewählt aus Biotin, den physiologisch verträglichen Salzen von Biotin, Biotinestern und den stereoisomeren Formen dieser Verbindungen
und
b) mindestens 1 Gew.-% einer oder mehrerer Verbindungen ausgewählt aus Harnstoff und Harnstoffderivaten, die nach Spaltung Harnstoff freisetzen.

2. Galenische Formulierung nach Anspruch 1, **dadurch gekennzeichnet, daß** der Anteil an der oder an den Verbindungen ausgewählt aus Biotin, den physiologisch verträglichen Salzen von Biotin, Biotinestern und den stereoisomeren Formen dieser Verbindungen von 0,05 bis 5 Gew.% bezogen auf die gesamte galenische Formulierung beträgt.

3. Galenische Formulierung nach einem der Ansprüche 1 oder 2, **dadurch gekennzeichnet, daß** sie Biotin enthält.

4. Galenische Formulierung nach einem oder mehreren der Ansprüche 1 bis 3, **dadurch gekennzeichnet, daß** der Anteil an der oder an den Verbindungen ausgewählt aus Harnstoff und Harnstoffderivaten von 1 bis 40 Gew.% bezogen auf die gesamte galenische Formulierung beträgt.

5. Galenische Formulierung nach einem oder mehreren der Ansprüche 1 bis 4, **dadurch gekennzeichnet, daß** sie Harnstoff enthält.

6. Galenische Formulierung nach einem oder mehreren der Ansprüche 1 bis 5, **dadurch gekennzeichnet, daß** sie neben einer oder mehreren Verbindungen ausgewählt aus Biotin, den physiologisch verträglichen Salzen von Biotin, Biotinestern und den stereoisomeren Formen dieser Verbindungen und neben einer oder mehreren Verbindungen ausgewählt aus Harnstoff und Harnstoffderivaten eine oder mehrere Substanzen mit Radikalfängereigenschaften enthält.

7. Galenische Formulierung nach einem oder mehreren der Ansprüche 1 bis 6, **dadurch gekennzeichnet, daß** sie in Form einer Lösung, einer Suspension, einer Emulsion, einer Paste, einer Salbe, eines Gels, einer Creme, einer Lotion, eines Lacks, eines Puders, einer Seife, eines tensidhaltigen Reinigungspräparates, eines Öls, eines Lippenstifts, eines Lippenpflegestifts, einer Mascara, eines Eyeliners, von Lidschatten, von Rouge, eines Puder-, Emulsions- oder Wachs-Makeups, eines Sonnenschutz-, Prä-Sun- und After-Sun-Präparats oder eines Sprays vorliegt.

8. Verwendung von
a) einer oder mehreren Verbindungen ausgewählt aus Biotin, den physiologisch verträglichen Salzen von Biotin, Biotinestern und den stereoisomeren Formen dieser Verbindungen
und
b) einer oder mehreren Verbindungen ausgewählt aus Harnstoff und Harnstoffderivaten
zur Herstellung einer topisch anwendbaren galenischen Formulierung nach Anspruch 1.

9. Verwendung nach Anspruch 8 zur Behandlung von Erkrankungen oder Funktionsstörungen menschlicher oder tierischer Haut, Haare und/oder Nägel.

10. Verwendung nach Anspruch 8 zur Therapie, Prophylaxe und/oder Metaphylaxe von Hauterkrankungen, von Funktionsstörungen der Haut, von Vorgängen der Hautalterung sowie von trockenen Hautzuständen.

11. Verwendung nach Anspruch 8 zur Verbesserung der Penetration der Verbindungen ausgewählt aus Biotin, den physiologisch unbedenklichen Salzen von Biotin, Biotinestern und den stereoisomeren Formen dieser Verbindungen in tiefere Schichten menschlicher oder tierischer Haut, Schleimhaut und/oder Nägel.

12. Verwendung nach Anspruch 8 zum Schutz, zur Erhaltung und/oder zum Wiederaufbau der normalen Funktion und Struktur menschlicher oder tierischer Haut und/oder zur Verhinderung umweltbedingter Hautschäden.

13. Verwendung nach Anspruch 8 zur Behandlung von Erkrankungen der Haare, Talg- und/oder Schweißdrüsen menschlicher oder tierischer Haut.

14. Verwendung nach Anspruch 8 zur kosmetischen Hautbehandlung.

15. Verfahren zur Herstellung einer topisch anwendbaren galenischen Formulierung nach Anspruch 1, **dadurch gekennzeichnet, daß** man
a) eine oder mehrere Verbindungen ausgewählt aus Biotin, den physiologisch verträglichen Salzen von Biotin, Biotinestern und den stereoisomeren Formen dieser Verbindungen
und
b) eine oder mehrere Verbindungen ausgewählt aus Harnstoff und Harnstoffderivaten
gegebenenfalls mit weiteren Wirkstoffen, Hilfs- und/oder Trägerstoffen in eine geeignete galenische Formulierungsform bringt.

## Claims

1. Galenic formulation comprising
a) one or more compounds selected from biotin, the physiologically tolerable salts of biotin, biotin esters and the stereoisomeric forms of these compounds
and
b) at least 1% by weight of one or more compounds selected from urea and urea derivatives which release urea after cleavage.

2. Galenic formulation according to Claim 1, **characterized in that** the proportion of the compound or of the compounds selected from biotin, the physiologically tolerable salts of biotin, biotin esters and the stereoisomeric forms of these compounds is from 0.05 to 5% by weight based on the total galenic formulation.

3. Galenic formulation according to one of Claims 1 or 2, **characterized in that** it contains biotin.

4. Galenic formulation according to one or more of Claims 1 to 3, **characterized in that** the proportion of the compound or of the compounds selected from urea and urea derivatives is from 1 to 40% by weight based on the total galenic formulation.

5. Galenic formulation according to one or more of Claims 1 to 4, **characterized in that** it contains urea.

6. Galenic formulation according to one or more of Claims 1 to 5, **characterized in that**, in addition to one or more compounds selected from biotin, the physiologically tolerable salts of biotin, biotin esters and the stereoisomeric forms of these compounds and in addition to one or more compounds selected from urea and urea derivatives, it contains one or more substances having free-radical scavenger properties.

7. Galenic formulation according to one or more of Claims 1 to 6, **characterized in that** it is present in the form of a solution, a suspension, an emulsion, a paste, an ointment, a gel, a cream, a lotion, a lake, a powder, a soap, a surfactant-containing cleansing preparation, an oil, a lipstick, a lipcare stick, mascara, an eyeliner, of eye shadow, of rouge, a powder, emulsion or wax make-up, a sunscreen, pre-sun and after-sun preparation or a spray.

8. Use of
a) one or more compounds selected from biotin, the physiologically tolerable salts of biotin, biotin esters and the stereoisomeric forms of these compounds
and
b) one or more compounds selected from urea and urea derivatives for the production of a topically applicable galenic formulation according to Claim 1.

9. Use according to Claim 8 for the treatment to diseases or functional disorders of human or animal skin, hair and/or nails.

10. Use according to Claim 8 for the therapy, prophylaxis and/or metaphylaxis of skin diseases, of functional disorders of the skin, of skin aging processes and of dry skin conditions.

11. Use according to Claim 8 for improving the penetration of the compounds selected from biotin, the physiologically acceptable salts of biotin, biotin esters and the stereoisomeric forms of these compounds into deeper layers of human or animal skin, mucous membrane and/or nails.

12. Use according to Claim 8 for the protection, for the maintenance and/or for the reconstruction of the normal function and structure of human or animal skin and/or for the prevention of environmentally related skin damage.

13. Use according to Claim 8 for the treatment of diseases of the hair, sebaceous and/or sweat glands of human or animal skin.

14. Use according to Claim 8 for cosmetic skin treatment.

15. Process for the production of a topically applicable galenic formulation according to Claim 1, **characterized in that**
a) one or more compounds selected from biotin, the physiologically tolerable salts of biotin, biotin esters and the stereoisomeric forms of these compounds
and
b) one or more compounds selected from urea and urea derivatives
are brought into a suitable galenic formulation form, if appropriate with further active compounds, excipients and/or vehicles.

## Revendications

1. Formulation galénique contenant :
a) un ou plusieurs composés choisis parmi la biotine, les sels de biotine acceptables sur le plan physiologique, les esters de biotine et les formes stéréoisomères de ces composés
et
b) au moins 1% en poids d'un ou plusieurs composés choisis parmi l'urée et les dérivés d'urée qui libèrent de l'urée après décomposition.

2. Formulation galénique selon la revendication 1, **caractérisée en ce que** la proportion du ou des composés choisis parmi la biotine, les sels de biotine acceptables sur le plan physiologique, les esters de biotine et les formes stéréoisomères de ces composés va de 0,05% à 5% en poids par rapport à la formulation galénique totale.

3. Formulation galénique selon l'une quelconque des revendications 1 ou 2, **caractérisée en ce qu'**elle contient de la biotine.

4. Formulation galénique selon l'une ou plusieurs des revendications 1 à 3, **caractérisée en ce que** la proportion du ou des composés choisis parmi l'urée et les dérivés d'urée va de 1% à 40% en poids par rapport à la formulation galénique totale.

5. Formulation galénique selon l'une ou plusieurs des revendications 1 à 4, **caractérisée en ce qu'**elle contient de l'urée.

6. Formulation galénique selon l'une ou plusieurs des revendications 1 à 5, **caractérisée en ce qu'**elle contient, en plus d'un ou plusieurs composés choisis parmi la biotine, les sels de biotine acceptables sur le plan physiologique, les esters de biotine et les formes stéréoisomères de ces composés, et en plus d'un ou plusieurs composés choisis parmi l'urée et les dérivés d'urée, une ou plusieurs substances ayant des propriétés de piège à radicaux libres.

7. Formulation galénique selon l'une ou plusieurs des revendications 1 à 6, **caractérisée en ce qu'**elle se présente sous la forme d'une solution, d'une suspension, d'une émulsion, d'une pâte, d'une pommade, d'un gel, d'une crème, d'une lotion, d'un vernis, d'une poudre, d'un savon, d'une préparation nettoyante contenant des tensio-actifs, d'une huile, d'un rouge à lèvres, d'un baume à lèvres, d'un mascara, d'un eye-liner, d'un fard à paupières, d'un fard à joues, d'un fond de teint en poudre, en émulsion ou en cire, d'une préparation pour protection solaire, avant-soleil et après-soleil, ou d'une pulvérisation.

8. Utilisation de
a) un ou plusieurs composés choisis parmi la biotine, les sels de biotine acceptables sur le plan physiologique, les esters de biotine et les formes stéréoisomères de ces composés
et
b) un ou plusieurs composés choisis parmi l'urée et les dérivés d'urée
pour la préparation d'une formulation galénique selon la revendication 1 destinée à une application topique.

9. Utilisation selon la revendication 8 pour le traitement de maladies ou de troubles fonctionnels de la peau, des poils et/ou des ongles chez l'Homme ou l'animal.

10. Utilisation selon la revendication 8 pour la thérapie, la prophylaxie et/ou la métaphylaxie de maladies de la peau, de troubles fonctionnels de la peau, de phénomènes de vieillissement cutané et d'états de sécheresse cutanée.

11. Utilisation selon la revendication 8 pour améliorer la pénétration des composés choisis parmi la biotine, les sels de biotine acceptables sur le plan physiologique, les esters de biotine et les formes stéréoisomères de ces composés dans les couches profondes de la peau, des muqueuses et/ou des ongles chez l'Homme ou l'animal.

12. Utilisation selon la revendication 8 pour protéger, conserver et/ou rétablir la fonction et la structure normales de la peau chez l'Homme ou l'animal et/ou pour prévenir des lésions cutanées dues à l'environnement.

13. Utilisation selon la revendication 8 pour le traitement de maladies du cuir chevelu, des glandes sébacées et/ou sudoripares de la peau chez l'Homme ou l'animal.

14. Utilisation selon la revendication 8 pour le traitement cosmétique de la peau.

15. Procédé de préparation d'une formulation galénique destinée à une administration topique selon la revendication 1, **caractérisée en ce que** l'on formule
a) un ou plusieurs composés choisis parmi la biotine, les sels de biotine acceptables sur le plan physiologique, les esters de biotine et les formes stéréoisomères de ces composés
et
b) un ou plusieurs composés choisis parmi l'urée et les dérivés d'urée
éventuellement avec d'autres substances actives, adjuvants et/ou véhicules, en une formulation galénique appropriée.
